# EUROPÄISCHE PATENTSCHRIFT

(11) **EP 0 758 447 B1**
(45) Veröffentlichungstag und Bekanntmachung des Hinweises auf die Patenterteilung: **04.11.1998**
(21) Anmeldenummer: 95919990.2
(22) Anmeldetag: 28.04.1995
(51) Int. Cl.: G01N 21/71

(54) **VERFAHREN UND VORRICHTUNG ZUR BESTIMMUNG VON ELEMENTZUSAMMENSETZUNGEN UND -KONZENTRATIONEN**
PROCESS AND DEVICE FOR DETERMINING ELEMENT COMPOSITIONS AND CONCENTRATIONS
PROCEDE ET DISPOSITIF POUR DETERMINER DES COMPOSITIONS ET DES CONCENTRATIONS D'ELEMENTS

(30) Priorität: 02.05.1994 DE 4415381
(43) Veröffentlichungstag der Anmeldung: 19.02.1997
(73) Patentinhaber: NIS Ingenieurgesellschaft mbH, D-63452 Hanau (DE)
(72) Erfinder: HNILICA, Klaus, D., D-63517 Rodenbach (DE); SCHNEIDER, Klaus, D-68309 Mannheim (DE)
(74) Vertreter: Hofstetter, Alfons J., Dr.rer.nat.
(86) Internationale Anmeldenummer: EP9501625
(87) Internationale Veröffentlichungsnummer: WO9530140

(56) Entgegenhaltungen:
- EP-A- 0 293 983
- EP-A- 0 476 728
- EP-A- 0 549 902
- WO-A-92/21957
- DE-A- 4 004 627
- DE-A- 4 138 157
- APPLIED SPECTROSCOPY., Bd.44, Nr.10, Dezember 1990, BALTIMORE US Seiten 1711 - 1714, XP169325 K.J. GRANT ET AL. 'time-resolved laser-induced breakdown spectroscopy of iron ore'

## Beschreibung

Die vorliegende Erfindung betrifft ein Verfahren zur Bestimmung von Elementzusammensetzungen und -konzentrationen von Materialproben, insbesondere der Konzentration bzw. des Vorhandenseins von Edelmetallen in unsortiertem Metallschrott, mittels lasergestützter Plasma-Emissions-Spektroskopie sowie einen hierzu geeigneten Meßkopf mit einer integrierten Strahloptik.

Der Nachweis oberflächennaher Elementkonzentrationen durch Spektralanalyse bei laserinduzierten Plasmen wird in der Literatur unter den Stichworten LIBS (= Laserinduzierte Breakdown Spektroskopie) und Laser-Spark-Spektroskopie diskutiert. Derartige Verfahren werden insbesondere zur Analyse von Metallteilchen verwendet. So beschreibt die deutsche Offenlegungsschrift DE 37 18 672 A1 ein Verfahren zur Analyse von nacheinander einzeln eine Prüfstrecke durchlaufenden Metallteilchen hinsichtlich ihrer Zusammensetzung. Dabei werden die Teilchen einem gepulsten Laserstrahl ausgesetzt, partiell zu Plasma verdampft und die Spektrallinien des Plasmas zur Identifizierung der Metallteilchen herangezogen. Bei diesem bekannten Verfahren wird aus der von dem Plasma ausgehenden Gesamtstrahlung ein bestimmter schmaler Wellenlängenbereich bzw. eine bestimmte Wellenlänge herausgefiltert. Nur die Intensität der herausgefilterten Teilstrahlung, d.h. nur eine bestimmte Wellenlänge bzw. ein Wellenlängenbereich, wird mit einem Vorbild verglichen, um eine qualitative Elementanalyse zu erhalten.

Ein in der deutschen Offenlegungsschrift DE 40 28 102 A1 beschriebenes Verfahren dient ebenfalls zur Analyse von Metallteilchen unterschiedlicher stofflicher Zusammensetzung. Mit Hilfe eines gepulsten Lasers wird unter Bildung eines Plasmas Metall von dem zu analysierenden Metall abgetragen. Aus dem Linienspektrum des Plasmas werden ebenfalls vorgegebene diskrete Spektrallinien oder Spektrallinienbereiche herausgefiltert. Dabei wird aus den Strahlungsintensitäten der Wellenlängen der herausgefilterten Spektrallinien bzw. Spektrallinienbereiche eine Kennzahl nach einem Algorithmus gebildet und durch den Vergleich dieser Kennzahl mit vorgegebenen Grenzwerten ein Sortiersignal gewonnen. Auch hierbei handelt es sich um eine qualitative Analyse der Metallteilchen.

Nachteilig an diesen bekannten Verfahren ist jedoch, daß es sich hierbei lediglich um qualitative Analyseverfahren handelt, deren Analysegenauigkeit niedrig ist. Bei den bekannten Verfahren ist eine hohe Analysegenauigkeit auch nicht notwendig, da hier lediglich ein Sortiersignal zur groben Aufteilung unterschiedlichster Metallteilchen zu bestimmten Elementgruppen erzeugt werden soll. Es hat sich aber gezeigt, daß eine lediglich qualitative Analyse von Materialien wie z.B. Metallteilchen bzw. Metallschrotte nicht mehr ausreicht, um eine wirtschaftlich sinnvolle Trennung und Wiederaufbereitung von z.B. Metallrohstoffen zu gewährleisten.

Das deutsche Patent DE 41 18 518 C2 beschreibt ein Verfahren zur Durchführung der Laser-Emissions-Spektroskopie. Dabei werden zusätzliche Messungen von Plasmaparametern wie Plasmatemperatur und Plasmadichte vorgenommen. Um eine quantitative Analyse zu gewährleisten, soll die Messung der Emissionsspektren nur bei definierten Plasmazuständen, die durch eine Beeinflussung der Intensität der plasmaerzeugenden Laserstrahlung erzielt wird, erfolgen. Nachteilig an diesem Verfahren ist, daß hierfür ein hoher konstruktiver und rechnerischem Aufwand zur Durchführung des Verfahrens notwendig ist, was die Störanfälligkeit des Verfahrens und der dafür notwendigen Vorrichtung deutlich erhöht.

APPLIED SPECTROSCOPY, Bd. 44, Nr. 10, Dez. 1990, p. 1711 ff. offenbart ein Verfahren der eingangs genannten Art, bei dem zur Verbesserung der Signalqualität über mehrere Pulse gemittelt wird.

Es ist daher Aufgabe der vorliegenden Erfindung, ein Verfahren der eingangs genannten Art bereitzustellen, welches eine einfache quantitative und qualitative Analyse mit hoher Analysegenauigkeit gewährleistet.

Weitere Aufgabe der Erfindung ist es, einen Meßkopf der eingangs genannten Art bereitzustellen.

Bekannte Vorrichtungen zur Bestimmung von Elementzusammensetzungen und -konzentrationen mittels der lasergestützten Plasma-Emissions-Spektroskopie weisen eine Laserlichtquelle zur Erzeugung gepulstem Laserstrahlen, einen oder mehrere Spektralfilter mit jeweils zugeordneten Spektral-Detektoren sowie Mittel zur elektronischen Auswertung der gewonnenen Spektralbilder auf. Nachteilig an diesen bekannten Vorrichtungen ist jedoch, daß die Laserquelle und die Spektrometer selbst die notwendigen optischen Einrichtungen wie Objektivsysteme enthalten. Damit wird allein durch die sich dadurch ergebende konstruktive Größe der Vorrichtungen deren Einsatzmöglichkeiten begrenzt.

Da die genannten Vorrichtungen mit relativ geringen Brennweiten arbeiten müssen, um den Energiebedarf des Lasers nicht zu groß werden zu lassen, ist es zudem notwendig, die Vorrichtungen möglichst nahe an die zu messenden Materialproben heranzuführen. Dabei muß bei den bekannten Vorrichtungen darauf geachtet werden, daß die integrierten empfindlichen Steuervorrichtungen für den Laser oder das Spektrometer keinen extremen physikalischen Bedingungen wie Temperatur und Druck ausgesetzt werden. Damit sind die Einsatzmöglichkeiten der bekannten Vorrichtungen zur Bestimmung von Elementzusammensetzungen und -konzentrationen von anorganischen Materialproben mittels lasergestützter Plasma-Emissions-Spektroskopie auch aus diesem Grund sehr eingeschränkt.

Aus der EP-A-549 902 ist ein Meßkopf für die Laserinduzierte Emissions spektroskopie bekannt, der über Lichtwellenleiter mit einem Laser und einem Analysator verbunden ist.

Desweiteren ist aus der deutschen Patentanmeldung DE 40 04 627 A1 eine Vorrichtung zur Durchführung eines Verfahrens zur Bestimmung von Materialeigenschaften polymerer Werkstoffe bekannt. Diese Vorrichtung weist Umlenkspiegel und Strahlteiler zur Führung eines Laserstrahls bzw. zur Rückführung der Plasmastrahlung zu einem Spektrographen auf.

Weiterhin ist aus der deutschen Patentanmeldung DE 41 38 157 A1 eine Vorrichtung zur Durchführung eines Verfahrens zur Bestimmung der Dicke einer Beschichtung mittels Laserstrahlung bekannt.

Um unabhängig von unterschiedlichen Oberflächenstrukturen der zu messenden Meterialproben zu werden, ist es notwendig, bei der Fokussierung des Laserstrahls mit relativ großen Brennweiten zu arbeiten, die - wie bereits erwähnt - eigentlich unerwünscht sind. Neben dem sich dadurch ergebenden erhöhten Energiebedarf ist es bei den bekannten Vorrichtungen nicht zu vermeiden, daß sich deutliche Variationen der Plasmaentstehungsbedingungen ergeben. Um derartige Variationen zu vermeiden, schlägt die deutsche Offenlegungsschrift DE 40 28 102 A1 vor, daß nur Metallteilchen unterhalb einer bestimmten Korngröße, wobei diese in einer Linie hintereinanderliegend vereinzelt an der Meßvorrichtung vorbeigeführt werden, analysiert werden sollen. Eine exakte Positionierung des Brennpunktes der bekannten Vorrichtung auf der Probenoberfläche ist dennoch nicht möglich, wodurch sich die genannten Variationen der Plasmaentstehungsbedingungen ergeben.

Es ist daher weitere Aufgabe der vorliegenden Erfindung, einen Meßkopf für eine Vorrichtung der eingangs genannten Art bereitzustellen, der eine exakte Positionierung des Brennpunktes des Laserstrahls auf der Probenoberfläche gewährleistet.

Zur Lösung der genannten Aufgaben dienen die Merkmale der unabhängigen Ansprüche.

Vorteilhafte Ausgestaltungen sind in den Unteransprüchen beschrieben.

Bei dem erfindungsgemäßen Verfahren wird u.a. zur Berechnung der Elementzusammensetzung und -konzentration von Materialproben das gesamte gemessene Emissicnsspektrum, d.h. die Summe einer Vielzahl von gemessenen Einzelspektren zugrundegelegt. Dadurch ergibt sich erfindungsgemäß eine signifikante Erhöhung der Analysegenauigkeit des Verfahrens. Dadurch kann sowohl eine qualitative wie auch quantitative Analyse durchgeführt werden.

Erfindungsgemäß wird dabei in einem ersten Verfahrensschritt a) ein Teil einer zu bestimmenden Materialprobe in einen plasmaartigen Zustand mittels eines auf die Materialprobe fokussierten, gepulsten Hochleistungslasers überführt. In einem weiteren erfindungsgemäßen Verfahrensschritt b) wird die vom Plasma emittierte Strahlung mittels eines Spektrometers spektral zerlegt. In einem dritten Verfahrensschritt c) wird das gesamte, sich jeweils pro Laserimpuls veränderte Emissionsspektrum in einem oder mehreren ausgewählten elementtypischen Spektralbereichen mittels eines Detektors gemessen. In einem abschließenden vierten Verfahrensschritt d) werden dann die Elementzusammensetzungen und -konzentrationen, wie oben dargelegt, berechnet.

In einer vorteilhaften Ausgestaltung des erfindungsgemäßen Verfahrens besteht der Hochleistungslaser aus einem frequenzvervielfachten Nd:YAG-Laser, der Strahlung mit einer Wellenlänge von 532 nm oder 355 nm aussendet. Durch Verwendung von Wellenlängen im sichtbaren oder ultravioletten Bereich ist es möglich, konstantere Emissionsspektren zu erhalten, wodurch die Analysegenauigkeit weiter verbessert und der Analysebereich optimiert wird. Zudem wurde festgestellt, daß bei der Verwendung eines Nd:YAG-Lasers mit diesen Wellenlängen keine Aufschmelzvorgänge auf der Materialprobenoberfläche stattfinden, die deutliche Variationen während der Plasmaentstehung im Plasma selbst verursachen könnten. Vielmehr wird die zu analysierende Materialprobe spontan verdampft, so daß sich nur minimale Variationen während der Plasmaentstehung ergeben, wodurch sich ebenfalls über die Gesamtdauer des Meßvorganges konstantere Emissionsspektren ergeben, so daß die Analysegenauigkeit des Verfahrens wiederum erhöht wird.

Erfindungsgemäß erfolgt die Fokussierung des Lasers gemäß Verfahrensschritt a) mittels Aufsetzen eines Meßkopfes auf die Materialprobe. Der Meßkopf umfaßt dabei ein Gehäuse, wobei innerhalb des Gehäuses in einem definierten, konstanten Abstand zu einer Austrittsöffnung des Gehäuses eine Strahloptik angeordnet ist, derart, daß der Brennpunkt des Laserstrahls in der Ebene der Austrittsöffnung liegt. Dadurch ist gewährleistet, daß unterschiedliche Oberflächenkonturen bzw. unterschiedliche Abmessungen des zu analysierenden Probenmaterials ausgeglichen werden. Durch den vordefinierten und konstanten Abstand zwischen der Austrittsöffnung des Gehäuses und der Strahloptik ergibt sich immer eine optimale Fokussierung des Laserstrahls. Auch ist dadurch vorteilhafterweise ein geringerer Fokusabstand möglich, wodurch der Laser mit niedrigeren Energieleistungen betrieben werden kann. Vorteilhafterweise beträgt die Pulsenergie des Lasers auf der Materialprobe 1 bis 10 mJ. Weiterhin ergibt sich der Vorteil, daß durch die exakte Fokussierungsmöglichkeit des Lasers Tiefenprofile der Elementzusammensetzung bzw. -konzentration der Materialprobe ermittelt werden können.

Gemäß der Erfindung weist der Meßkopf mindestens ein bewegliches Abstandselement auf, wobei das Abstandselement in einer ersten Position teilweise aus dem der zu messenden Probe zugewandten Seite des Gehäuses herausragt und in einer zweiten Position sich innerhalb des Gehäuses befindet. In der zweiten Position gibt das Abstandselement eine Auslösevorrichtung für die Laserquelle frei. Dadurch ist gewährleistet, daß erst bei dem Aufsetzen des Meßkopfes auf die Materialprobe und damit der Fokussierung des Lasers dieser in Betrieb gesetzt wird. Damit ist wiederum gewährleistet, daß sich lediglich minimale Variationen der Plasmaentstehungsbedingungen während des Meßvorganges bzw. der Meßvorgänge ergeben.

In einer weiteren vorteilhaften Ausgestaltung der Erfindung besteht der im Verfahrensschritt c) verwendete Detektor aus einem Vielkanal-Detektor. Der Vielkanaldetektor kann dabei aus Photodioden-Arrays oder CCD-Arrays oder aus mit Photomultipliern bestückten Zeilen bestehen. Damit ist vorteilhafterweise ein simultaner Nachweis des gesamten spektralzerlegten Plasmaemissionsspektrums möglich.

In einer weiteren vorteilhaften Ausgestaltung des erfindungsgemäßen Verfahrens wird im Verfahrensschritt c) eine zeitliche Filterung der Plasmaemissionen mittels eines optischen Transmissionsschalters durchgeführt. Die Filterung besteht dabei aus einem Ausblenden des breitbandigen Emissionshintergrundes in einem Zeitraum von 0 - 500 nsec nach einem Laserpuls und/oder einem Ausblenden von Molekül- und/oder Clusterspektren im µsec-bis msec-Bereich nach einem Laserpuls. Dadurch ist vorteilhafterweise gewährleistet, daß einerseits die Analysegenauigkeit erhöht werden kann und andererseits die zu verarbeitende Datenmenge reduziert wird.

Die Auswertung des gesamten gemessenen Emissionsspektrums gemäß Verfahrensschritt d) kann mit verschiedenen vorteilhaften Berechnungsmethoden durchgeführt werden. So kann die Berechnung der Elementzusammensetzungen und -konzentrationen einerseits mit Hilfe mathematischer Korrelationsverfahren unter Verwendung eines oder mehrerer Kalibrierspektren einer oder mehrerer Kalibrierproben mit bekannter Element zusammensetzung und -konzentration erfolgen. Vorteilhafterweise wird dabei ein Kreuzkorrelationsverfahren angewendet. Andererseits kann diese Berechnung auch mittels einer Integration mehrerer Einzelmessungen bzw. -spektren durchgeführt werden, wobei eine Mittelung der Puls-zu-Puls-Variationen der Einzelspektren während der Plasmaerzeugung erfolgt. Diese Integration kann dabei vorteilhafterweise im Detektor erfolgen. Dadurch ist ebenfalls eine Reduktion des zu verarbeitenden Datenflusses gewährleistet, wodurch Laser mit einer sehr hohen Wiederholrate bzw. Impulsrate einsetzbar sind. Die Integration der Einzelmessungen kann jedoch auch mit Hilfe einer dem Detektor nachgeschalteten Datenverarbeitungsanlage unter Verwendung entsprechender Software erfolgen. Eine erfindungsgemäße Erhöhung der Analysegenauigkeit ergibt sich aber auch durch die Verwendung der Gesamtheit oder einer Teilmenge elementtypischer Spektrallinien für die Berechnung der Konzentrationen einzelner Elemente.

Bei einer Vorrichtung zur Bestimmung von Elementzusammensetzungen und -konzentrationen von Materialproben ist der Hochleistungslaser und das Spektrometer von dem Meßkopf erfindungsgemäß geometrisch und räumlich entkoppelt, wobei der Meßkopf über mindestens einen Lichtwellenleiter mit dem Laser und/oder dem Spektrometer verbunden ist. Durch die Entkoppelung von Laser, Spektrometer und Meßkopf können die empfindlichen Laser- und Spektrometervorrichtungen räumlich anders positioniert werden als der auf die zu analysierende Materialprobe aufzusetzende Meßkopf. Dadurch ist gewährleistet, daß der Meßkopf bei entsprechender Ausgestaltung, z.B. durch geeignete Materialwahl, bei extremen Temperatur- oder Druckbedingungen eingesetzt werden kann, ohne daß es zu einer Schädigung der Laserquelle bzw. der Lasersteuervorrichtung oder des Spektrometers kommt.

Durch den Lichtwellenleiter ist zudem vorteilhafterweise ein verlustarmer Transport der Plasmaemissionen zu einem Spektrometer gewährleistet. Durch den Einsatz von Lichtwellenleitern ist es weiterhin möglich, eine simultane Messung an mehreren unterschiedlichen Meßpositionen durch Aufspaltung des Laserstrahls in mehrere Lichtwellenleiter vorzunehmen. Vorteilhafterweise erhöht sich dadurch die Analysegeschwindigkeit.

Mindestens ein Lichtwellenleiter dient zur Übertragung der Plasmaemissionen zu dem Spektrometer. Durch die Fokussierung der Plasmaemissionen auf den zweiten Lichtwellenleiter mit der gleichen Strahloptik, welche für die Fokussierung des Lasers verwendet wird, ist ein einfacher und robuster Aufbau des Meßkopfes möglich. Durch die Anordnung mehrerer Lichtwellenleiter zur Übertragung der Plasmaemissionsspektren an der Strahloptik ergibt sich weiterhin der Vorteil, daß dadurch die Meßzeit für eine Einzelmessung minimiert wird.

In einer weiteren vorteilhaften Ausgestaltung besteht der Hochleistungslaser aus einem frequenzvervielfachten Nd:YAG-Laser. Die vorteile dieses Lasertyps wurden bereits beschrieben. Gleiches gilt für die Verwendung von einem Vielkanal-Detektor in der erfindungsgemäßen Vorrichtung.

Weiterhin wird erfindungsgemäß ein Meßkopf für eine derartige Vorrichtung vorgeschlagen, der aus einer in einem Gehäuse angeordneten Strahloptik besteht, wobei das Gehäuse mindestens ein bewegliches Abstandselement aufweist. Das Abstandselement ragt dabei in einer ersten Position teilweise aus dem der zu messenden Probe zugewandten Seite des Gehäuses heraus und befindet sich in einer zweiten Position vollständig innerhalb des Gehäuses. In dieser zweiten Position gibt das Abstandselement eine Auslösevorrichtung für den Laser frei. Dadurch ist gewährleistet, daß der Laserpuls erst mit dem Aufsetzen des eigentlichen Gehäuses des Meßkopfes und damit der exakten Positionierung und Fokussierung des Laserstrahls erfolgt. Somit können Unebenheiten in der Oberfläche der zu messenden Materialprobe ausgeglichen werden.

In einer weiteren vorteilhaften Ausgestaltung ist die Strahlenoptik in einem definierten, konstanten Abstand zu einer Austrittsöffnung des Gehäuses angeordnet, so daß der Brennpunkt des Laserstrahls in der Ebene der Austrittsöffnung liegt. Durch diese Anordnung ergibt sich vorteilhafterweise, daß immer mit einer konstanten Brennweite fokussiert und gearbeitet werden kann, wodurch die Pulsenergie des Lasers auf der Materialprobe verringert werden kann. Üblicherweise betragen die Pulsenergien des Lasers auf der Materialprobe 1 - 10 mJ. Die Strahloptik ist vorteilhafterweise in einem Abstand von 25 - 40 mm zu der Austrittsöffnung des Gehäuses angeordnet.

Weitere Einzelheiten, Merkmale und Vorteile der Erfindung ergeben sich aus der folgenden Beschreibung von zeichnerisch dargestellten Ausführungsbeispielen. Es zeigen:
- Figuren 1a - 1d: vier gemessene Einzelspektren einer anorganischen Materialprobe (Stahlprobe);
- Figur 2: ein gesamtes gemessenes Emissionsspektrum von 30 gemittelten Einzelspektren;
- Figur 3: eine schematische Darstellung der erfindungsgemäßen Vorrichtung;
- Figur 4: eine schematische Darstellung eines Meßkopfes der erfindungsgemäßen Vorrichtung; und
- Figur 5: eine schematische Darstellung einer weiteren Ausführungsform eines Meßkopfes der erfindungsgemäßen Vorrichtung.

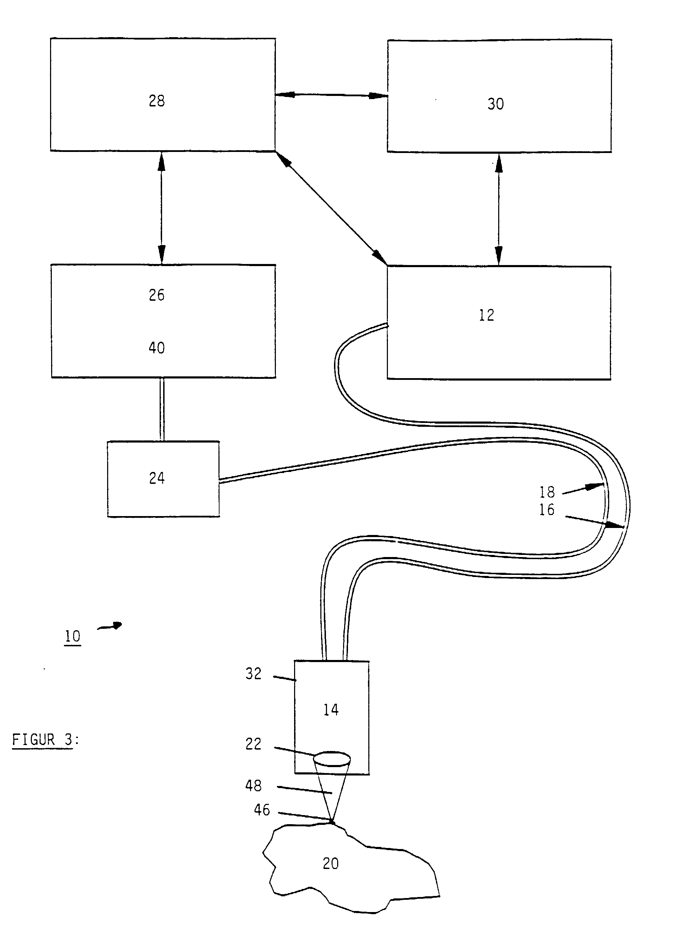

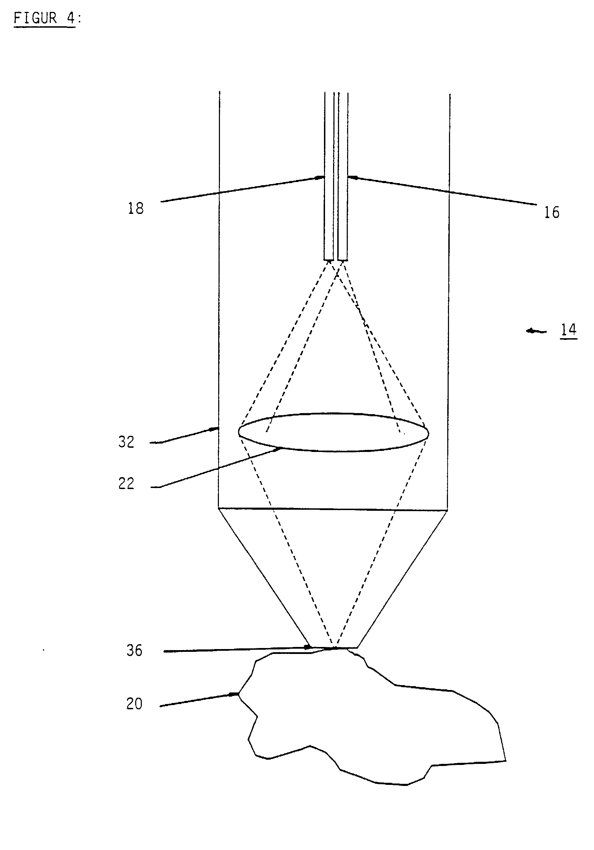

Gemäß Figur 3 wird eine Materialprobe 20, nämlich eine Stahlprobe, von einem Laser 12 mit Laserstrahlung 48 bestrahlt. Dabei wird gemäß dem ersten erfindungsgemäßen Verfahrensschritt a) ein Teil der Materialprobe 20 in einen plasmaartigen Zustand 46 überführt. Der Hochleistungslaser 12 ist dabei gepulst und besteht aus einem frequenzvervielfachten Nd:YAG Laser. Die Anregungsstrahlung des Nd:YAG-Lasers weist dabei eine Wellenlänge von 1064 nm oder kürzere Wellenlängen (bei Frequenzvervielfachung) von 532 nm oder 355 nm auf. Die Pulsenergie des Lasers 12 auf der Materialprobe 20 beträgt dabei 1 - 10 mJ. Die Pulsrate des Lasers 12 beträgt 10 - 500 Hz, vorzugsweise 30 - 100 Hz. Die Pulsdauer beträgt typischerweise 10 nsec. Die Fokussierung des Lasers 12 bezüglich der Materialprobe 20 erfolgt mittels Aufsetzen eines Meßkopfes 14 (vgl. Figur 4) auf die Materialprobe 20. Gemäß Figur 4 umfaßt der Meßkopf 14 ein Gehäuse 32, wobei innerhalb des Gehäuses 14 in einem definierten, konstanten Abstand zu einer Austrittsöffnung 38 des Gehäuses 32 eine Strahloptik 22 angeordnet ist, derart, daß der Brennpunkt des Laserstrahls in der Ebene der Austrittsöffnung 38 liegt. Dadurch ergibt sich eine exakte Positionierung des Brennpunktes auf der Probenoberfläche, wodurch die Variationen der Bedingungen bei der Plasmaentstehung minimiert werden können. Zudem ergibt sich eine Minimierung des Abstandes des Meßkopfes 14 von der Probe 20, so daß dadurch auch eine Minimierung der erforderlichen Laserenergie erfolgt. Typischerweise werden Laserenergien im Bereich von 5 mJ eingesetzt.

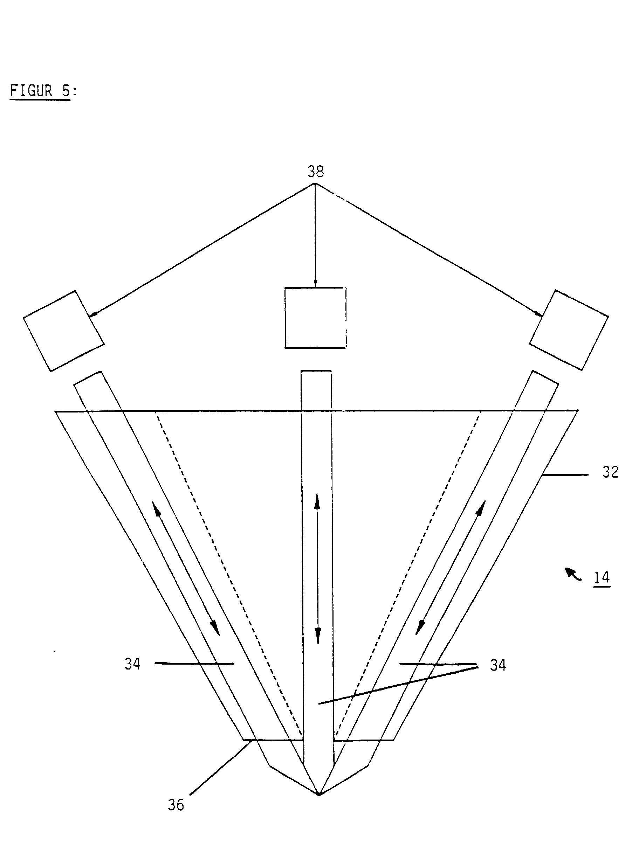

Um die exakte Positionierung und Fokussierung des Lasers 12 auf die Materialprobe 20 sicherzustellen und gleichzeitig zu verhindern, daß ein Laserpuls vor dem endgültigen Aufsetzen des Meßkopfes 14 auf die Materialprobe 20 erfolgt, weist der Meßkopf 14 in einem in Figur 5 dargestellten Ausführungsbeispiel mindestens ein im oder am Gehäuse 32 angeordnetes bewegliches Abstandselement 34 auf. Das Abstandselement 34 ragt dabei in einer ersten Position teilweise aus dem der zu messenden Probe 20 zugewandten Seite des Gehäuses 32 heraus und befindet sich in einer zweiten Position vollständig innerhalb des Gehäuses 32. Erst, wenn sich das Abstandselement 34 in der zweiten Position befindet, gibt es eine Auslösevorrichtung 38 für den Laser 12 frei.

In einem zweiten erfindungsgemäßen Verfahrensschritt b) erfolgt die spektrale Zerlegung einer vom Plasma 46 emittierten Strahlung mit Hilfe eines in Figur 3 dargestellten Spektrometers 26.

In einem weiteren erfindungsgemäßen Verfahrensschritt c) wird das gesamte, sich jeweils pro Laserimpuls verändernde Emissionsspektrum 44 des Plasmas 46 in einem oder mehreren Spektralbereichen mittels eines in Figur 3 dargestellten Detektors 40 gemessen. Der Detektor 40 besteht dabei aus einem Vielkanal-Detektor. Durch die Verwendung eines Vielkanal-Detektors ist ein simultaner Nachweis des gesamten spektral zerlegten Plasmaemissionsspektrums möglich. Als Detektoren 40, können Photodioden-Arrays, CCD-Arrays, mit Photomultipliern bestückte Zeilen oder vergleichbare Detektoren verwendet werden.

Im Verfahrensschritt c) kann zusätzlich eine zeitliche Filterung der Plasmaemissionen mittels eines optischen Transmissionsschalters 24 durchgeführt werden. Die Filterung besteht dabei aus einem Ausblenden des breitbandigen Emissionsuntergrundes in einem Zeitraum von 0 - 500 nsec nach einem Laserpuls und/oder einem Ausblenden von Molekül- und/oder Clusterspektren in µsec bis msec-Zeitbereich nach einem Laserpuls. Durch diese Filterung ergibt sich eine Datenreduzierung, was die Digitalisierung und Speicherung des gemessenen Spektrums erleichtert und beschleunigt.

In einem weiteren erfindungsgemäßen Verfahrensschritt d) wird die Element zusammensetzung und -konzentration der gemessenen Materialprobe 20 unter Zugrundelegung des gesamten Emissionsspektrums 44 berechnet. Bei dem gesamten gemessenen Emissionsspektrum 44 handelt es sich um die Summe einer Vielzahl von gemessenen Einzelspektren 42. Um die gewünschte quantitative Analysegenauigkeit zu erreichen, werden mindestens 5, vorzugsweise 10 - 100 Einzelspektren 42 ausgewertet, d.h. zu einem Gesamtspektrum 44 zusammengefaßt.

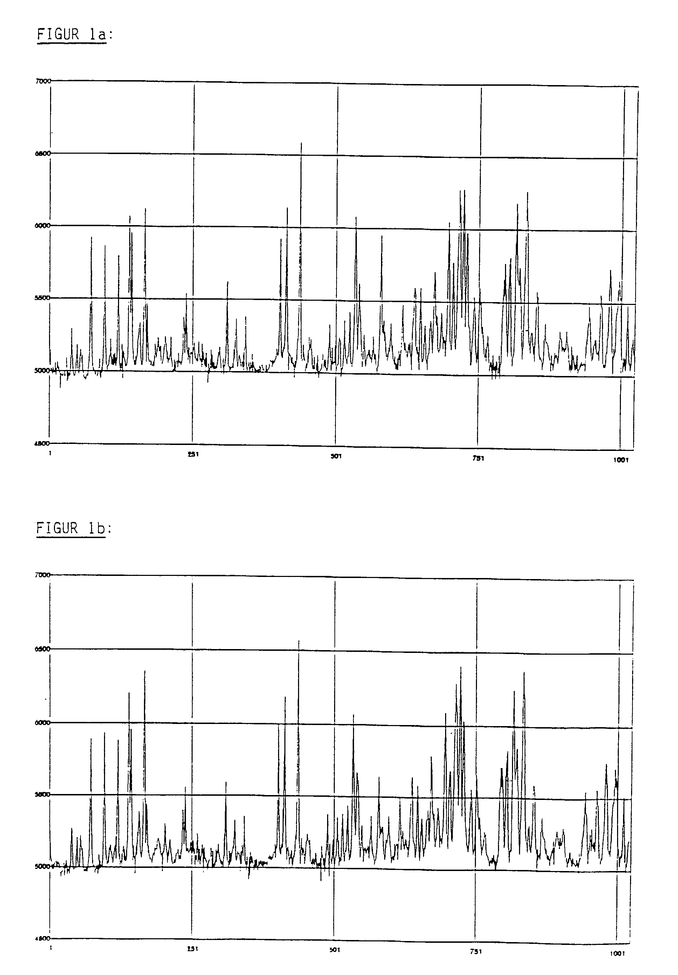

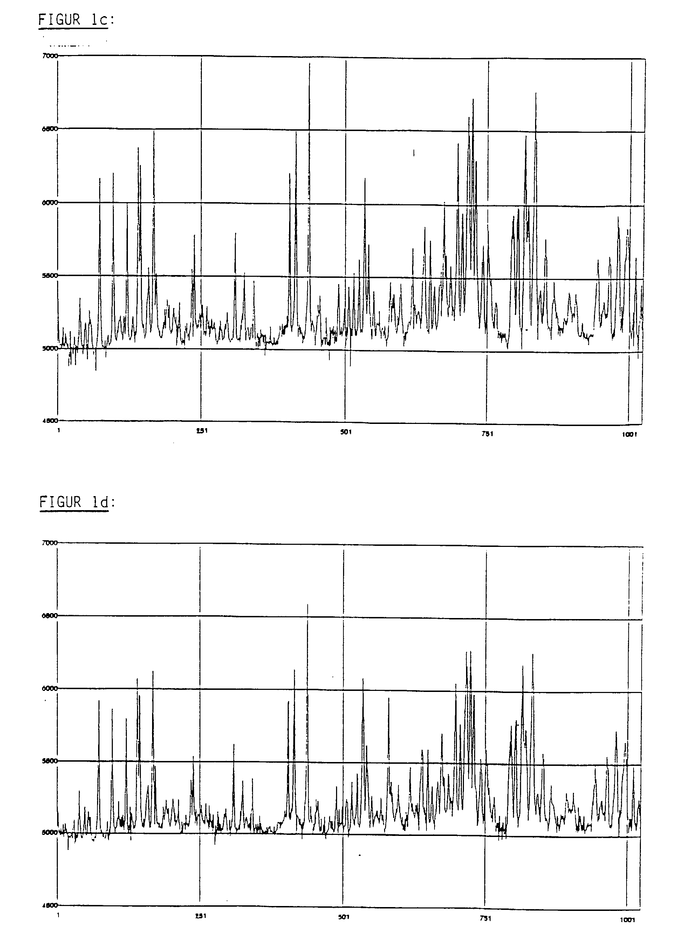

Die Figuren 1a - 1d zeigen vier gemessene Einzelspektren 42 der anorganischen Materialprobe 20 (Stahlprobe). Dabei sind auf der Ordinate die Intensitäten der Emissionsstrahlung gegen die entsprechende Wellenlänge auf der Abszisse abgetragen. Das Emissionsspektrum umfaßt dabei einen Wellenlängenbereich von ca. 45 nm. Ein Vergleich der zu unterschiedlichen Zeitpunkten aufgenommenen Emissionsspektren der gleichen Materialprobe zeigt, daß die Einzelspektren signifikante Unterschiede in den Intensitäten bei einzelnen Wellenlängen aufweisen, insgesamt jedoch relativ konstante Plasmabildungsbedingungen widerspiegeln.

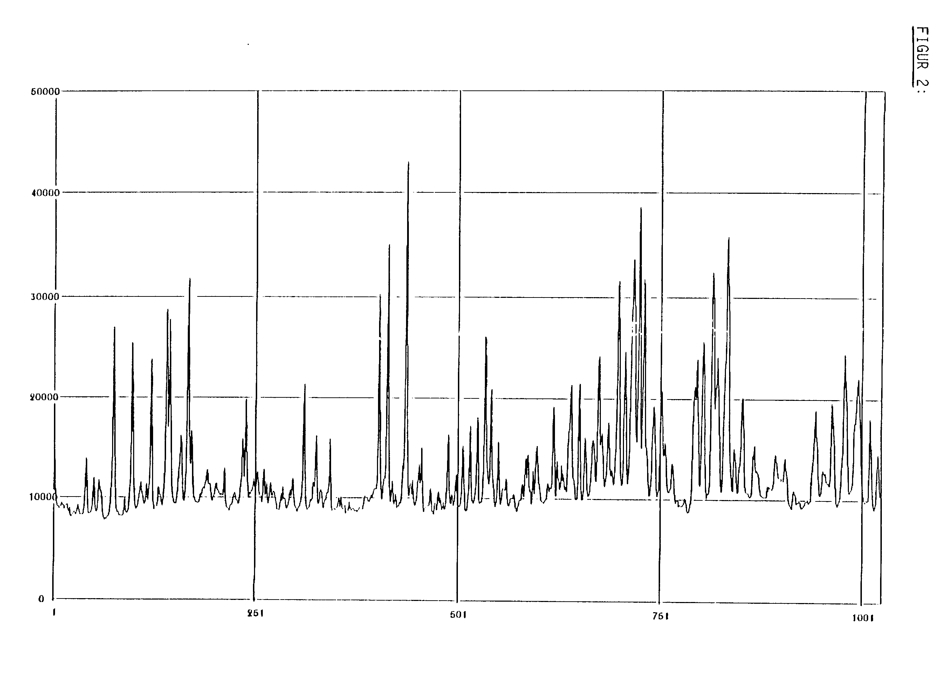

Figur 2 zeigt ein gesamtes gemessenes Emissionsspektrum 44 der gleichen Materialprobe 20, welches der Berechnung der Elementzusammensetzung und -konzentration gemäß dem erfindungsgemäßen Verfahrensschritt d) zugrundegelegt wird. Das dargestellte Gesamtemissionsspektrum 44 ergibt sich durch die Integration von 30 Einzelmessungen bzw. -spektren 42, wobei eine Mittelung der Puls-zu-Puls-Variationen der Einzelemissionsspektren 42 während der Plasmaerzeugung erfolgt. Damit ergibt sich eine deutliche Erhöhung der Analysegenauigkeit. Die Integration der Einzelmessungen 42 kann dabei im Detektor 40 erfolgen. Dies bedeutet, daß vor dem Auslesen der Detektordaten diese integriert werden und somit nur ein reduzierter Datenfluß der Auswertung in einer nachgeschalteten Datenverarbeitungsanlage 28 unter Verwendung entsprechender Software erfolgt. Durch die Reduktion des Datenflusses können Laser mit sehr hoher Wiederholrate eingesetzt werden. Insbesondere betrifft dies Laser mit Impulsraten >30 Hz. Es ist jedoch auch möglich, die Integration der Einzelmessungen direkt in der dem Detektor nachgeschalteten Datenverarbeitungsanlage 28 zu vollziehen.

Zur gewünschten Erhöhung der Analysegenauigkeit ist es aber auch möglich, die Berechnung der Elementzusammensetzungen und -konzentrationen gemäß Verfahrensschritt d) mittels mathematischer Korrelationsverfahren unter Verwendung eines oder mehrerer Kalibrierspektren einer oder mehrerer Kalibrierproben mit bekannter Elementzusammensetzung und -konzentration durchzuführen. Vorzugsweise wird dabei ein Kreuzkorrelationsverfahren angewendet. Es ist jedoch auch denkbar, andere geeignete spezielle Korrelationsverfahren für die Auswertung des gemessenen Gesamtspektrums 44 zu verwenden.

Figur 3 zeigt eine schematische Darstellung einer Vorrichtung 10 zur Bestimmung von Elementzusammensetzungen und -konzentrationen von Materialproben. Die Vorrichtung 10 umfaßt dabei einen Laser 12, ein Spektrometer 26 und einen Meßkopf 14 mit einer integrierten Strahloptik 22. Man erkennt, daß der Hochleistungslaser 12 und das Spektrometer 26 von dem Meßkopf 14 geometrisch und räumlich entkoppelt sind. Der Meßkopf 14 ist über einen Lichtwellenleiter 16 mit dem Laser 12 verbunden. Mit dem Spektrometer 26 ist der Meßkopf 14 über einen zweiten Lichtwellenleiter 18 verbunden. Es erfolgt eine Einkoppelung des Laserstrahls von dem Laser bzw. der Laserquelle 12 in den Lichtwellenleiter 16. Durch den Lichtwellenleiter 16 ist ein verlustarmer Transport des Laserstrahls zu dem Meßkopf 14 möglich. Eine typische Dämpfung des Laserstrahls beträgt ca. 1 Größenordnung je km Transportlänge. Der Hochleistungslaser 12 besteht vorzugsweise aus einem frequenzvervielfachten Nd:YAG-Laser. Es können jedoch auch andere geeignete Lasertypen verwendet werden. Zudem umfaßt die dargestellte Vorrichtung 10 einen zwischen der Strahloptik 22 und dem Spektrometer 26 im Weg des Lichtwellenleiters 18 angeordneten Transmissionsschalter 24. Der Transmissicnsschalter 24 besteht dabei aus einer Pockelszelle. Im Spektrometer 26 ist ein Detektor 40, nämlich ein Vielkanal-Detektor, ausgebildet. Der Detektor 40 kann dabei aus Photodioden-Arrays, CCD-Arrays oder aus mit Photomultipliern bestückten Zeilen bestehen. Auch können andere Detektoren verwendet werden, die vergleichbare Eigenschaften aufweisen.

Die Datenverarbeitungsanlage 23 dient einerseits zur Auswertung der gemessenen Gesamtspektren 44 bzw. der Berechnung der Elementzusammensetzungen und -konzentrationen, andererseits erfolgt mit Hilfe der Datenverarbeitungsanlage 28 die Steuerung des Lasers 12. Diese Steuerung kann direkt erfolgen, kann aber auch über eine mit der Datenverarbeitungsanlage 28 verbundene, getrennt angeordnete Lasersteuervorrichtung 30 erfolgen. Zudem ist es möglich, die Datenverarbeitungsanlage 28 zur Steuerung automatisierter Positioniersysteme für die Positionierung des Meßkopfes 14 einzusetzen.

In einer nicht dargestellten Ausführungsform können mehrere Meßköpfe 14 mit dem Laser 12 über mehrere Lichtwellenleiter 16 verbunden sein. Die dadurch erreichte Aufspaltung des Laserstrahls ermöglicht eine simultane Messung an mehreren unterschiedlichen Meßpositionen. Dies wird erst durch das erfindungsgemäße Verfahren ermöglicht, da hierfür nur minimale Laserenergien pro Laserposition benötigt werden. Es ist aber auch denkbar, mehrere Lichtwellenleiter 18 für die Übertragung der Plasma-Emissions-Spektren von einem oder mehreren Meßköpfen 14 zum Spektrometer 26 zu verwenden. Bei gleichzeitiger Verwendung mehrerer Vielkanaldetektoren 40 oder eines Vielkanaldetektors mit einer flächigen, zweidimensionalen Anordnung von Einzeldetektoren ergibt sich eine deutliche Minimierung der Meßzeit für jede Einzelmessung.

Die dargestellte Vorrichtung 10 ist durch die räumliche Trennung von empfindlichen Elementen der Vorrichtung 10, wie der Laserquelle 12 oder dem Spektrometer 26, über einen oder mehrere Lichtwellenleiter 16, 18, für den Einsatz bei rauhen Umgebungsbedingungen, z.B. in Stahlwerken auf Schrottplätzen, im Freien, in Nuklearbereichen geeignet.

Figur 4 zeigt eine schematische Darstellung des Meßkopfes 14 der Vorrichtung 10. Man erkennt, daß im Meßkopf 14 eine Aufweitung des Laserstrahls und eine anschließende starke Fokussierung mit Hilfe der Strahloptik 22 erfolgt. Die Strahloptik 22 kann dabei aus einer einzelnen Linse oder aus einem Linsensystem bestehen. Die Fokussierung des Laserstrahles erfolgt derart, daß der Brennpunkt des Laserstrahls in der Ebene einer Austrittsöffnung 36 liegt. Typische Brennweiten betragen zwischen 25 und 40 mm. Da die Strahloptik 22 in einem definierten, konstanten Abstand zu der Austrittsöffnung 36 des Gehäuses 32 angeordnet ist, erfolgt die Fokussierung nach Aufsetzen des Meßkopfes 14 auf die Materialprobe 20 immer mit der gleichen konstanten Brennweite.

Figur 5 zeigt eine schematische Darstellung einer Ausführungsform des Meßkopfes 14 der Vorrichtung 10. Der Meßkopf 14 umfaßt dabei mindestens ein bewegliches Abstandselement 34, welches in oder an dem Gehäuse 32 angeordnet ist. Das Abstandselement 34 ragt dabei in einer ersten Position (wie in der Figur dargestellt) teilweise aus der der zu messenden Probe 20 zugewandten Seite 32 heraus. Die in dem Ausführungsbeispiel dargestellten drei Abstandselemente berühren sich in dieser ersten Position gegenseitig, so daß eine Art Verschluß der Austrittsöffnung 36 entsteht. In einer zweiten Position befinden sich die Abstandselemente 34 vollständig innerhalb des Gehäuses 32. Dabei geben die Abstandselemente eine Auslösevorrichtung 38 für den Laser 12 frei. Die Freigabe kann dabei mechanisch, elektronisch oder auf jede andere geeignete Weise erfolgen.

## Patentansprüche

1. Meßkopf mit einem Gehäuse (32) geeignet zum Einsatz in einer Vorrichtung zur Bestimmung von Elementzusammensetzungen und -konzentrationen von Materialproben, insbesondere der Konzentration bzw. des Vorhandenseins von Edelmetallen in unsortiertem Metallschrott, mittels lasergestützter Plasmaemissionsspektroskopie mit einem Hochleistungslaser (12) und einem Spektrometer (26), wobei der Hochleistungslaser (12) und das Spektrometer (26) von dem ein eigenes Gehäuse (32) aufweisenden Meßkpof (14) geometrisch und räumlich entkoppelt sind, wobei der Meßkopf (14) eine integrierte Strahloptik (22) aufweist und der Meßkopf (14) bzw. die integrierte Strahloptik (22) über mindestens einen, im Meßkopf (14) angeordneten Lichtwellenleiter (16, 18) mit dem Laser (12) und/oder dem Spektrometer (26) verbunden ist,
**dadurch gekennzeichnet,**
daß das Gehäuse (32) mindestens ein bewegliches Abstandselement (34) aufweist, wobei das Abstandselement (34) in einer ersten Position teilweise aus der der zu messenden Probe (20) zugewandten Seite des Gehäuses (32) herausragt und in einer zweiten Position sich vollständig innerhalb des Gehäuses (32) befindet, wobei in der zweiten Position das Abstandselement (34) eine Auslösevorrichtung (38) für den Laser (12) freigibt.

2. Meßkopf nach Anspruch 1,
**dadurch gekennzeichnet,**
daß die Strahloptik (22) in einem definierten, konstanten Abstand zu einer Austrittsöffnung (36) des Gehäuses (32) angeordnet ist, so daß der Brennpunkt des Laserstrahls in der Ebene der Austrittsöffnung (36) liegt.

3. Meßkopf nach Anspruch 2,
**dadurch gekennzeichnet,**
daß die Strahloptik (22) einen Abstand von 25 - 40 mm zu der Austrittsöffnung (36) des Gehäuses (32) aufweist.

4. Meßkopf nach Anspruch 1,
**dadurch gekennzeichnet,**
daß der Hochleistungslaser (12) aus einem frequenzvervielfachten Nd:YAG-Laser besteht.

5. Meßkopf nach Anspruch 1 oder 4,
**dadurch gekennzeichnet,**
daß zwischen dem Spektrometer (26) und der Strahloptik (22) ein Transmissionsschalter (24) angeordnet ist.

6. Meßkopf nach Anspruch 5,
**dadurch gekennzeichnet,**
daß der Transmissionsschalter (24) aus einer Pockelszelle besteht.

7. Meßkopf nach einem der Ansprüche 1 oder 4 bis 6,
**dadurch gekennzeichnet,**
daß in dem Spektrometer (26) ein Detektor (40) ausgebildet ist.

8. Meßkopf nach Anspruch 7,
**dadurch gekennzeichnet,**
daß der Detektor (40) aus einem Vielkanal-Detektor besteht.

9. Meßkopf nach Anspruch 7 oder 8,
**dadurch gekennzeichnet,**
daß der Detektor (40) aus Photodioden-Arrays oder CCD-Arrays oder aus mit Photomultipliern bestückten Zeilen besteht.

10. Verfahren zur Bestimmung von Elementzusammensetzungen und - konzentrationen von Materialproben, insbesondere der Konzentration bzw. des Vorhandenseins von Edelmetallen in unsortiertem Metallschrott, mittels lasergestützter Plasmaemissionsspektroskopie, wobei das Verfahren die Schritte
a) Überführung eines Teils der zu bestimmenden Materialprobe (20) in einen plasmaartigen Zustand (46) mittels eines auf die Materialprobe (20) fokussierten, gepulsten Hochleistungslasers (12);
b) spektrale Zerlegung einer vom Plasma (46) emittierten Strahlung mittels eines Spektrometers (26);
c) Messung von Einzelspektren (42) des Plasmas (46) bei verschiedenen Laserimpulsen in einem oder mehreren elementtypischen Spektralbereichen mittels eines Vielkanaldetektors (40),
d) Berechnung der Elementzusammensetzung und -konzentration der Materialproben unter Zugrundelegung des gesamten gemessenen Emissionsspektrums (44), d.h. der Summe einer Vielzahl von gemessenen Einzelspektren (42), wobei die Berechnung mittels einer Integration mehrerer Einzelmessungen bzw. -spektren (42) durchgeführt wird und wobei eine Mittelung der Puls-zu-Puls-Variationen der Einzelspektren (42) während der Plasmaerzeugung erfolgt und die Integration der Einzelmessungen im Vielkanaldetektor (40) durchgeführt wird,
umfaßt und die Fokussierung des Lasers (12) gemäß Verfahrensschritt a) mittels Aufsetzen eines Meßkopfes (14) auf die Materialprobe (20) erfolgt, wobei der Meßkopf (14) ein Gehäuse (32) umfaßt und innerhalb des Gehäuses (14) in einem definierten, konstanten Abstand zu einer Austrittsöffnung (38) des Gehäuses (32) eine Strahloptik (22) angeordnet ist, derart, daß der Brennpunkt des Laserstrahls in der Ebene der Austrittsöffnung (38) liegt,
**dadurch gekennzeichnet,**
daß das Gehäuse (32) mindestens ein bewegliches Abstandselement (34) aufweist, wobei das Abstandselement (34) in einer ersten Position teilweise aus der der zu messenden Probe (20) zugewandten Seite des Gehäuses (32) herausragt und in einer zweiten Position sich vollständig innerhalb des Gehäuses (32) befindet, wobei in der zweiten Position das Abstandselement (34) eine Auslösevorrichtung (38) für die Laserquelle (12) freigibt.

11. Verfahren nach Anspruch 10,
**dadurch gekennzeichnet,**
daß im Verfahrensschritt c) eine zeitliche Filterung der Plasmaemissionen mittels eines optischen Transmissionsschalters (24) durchgeführt wird.

12. Verfahren nach Anspruch 11,
**dadurch gekennzeichnet,**
daß die Filterung aus einem Ausblenden des breitbandigen Emissionsuntergrundes in einem Zeitraum von 0 - 500 nsec nach einem Laserpuls und/oder einem Ausblenden von Molekül- und/oder Clusterspektren im µsec- bis msec-Zeitbereich nach einem Laserpuls besteht.

13. Verfahren nach einem der Ansprüche 10 bis 12,
**dadurch gekennzeichnet,**
daß im Verfahrensschritt d) mindestens 5, vorzugsweise 10 - 100 Einzelspektren (42) ausgewertet werden.

14. Verfahren nach einem der Ansprüche 10 bis 13,
**dadurch gekennzeichnet,**
daß die Berechnung der Elementzusammensetzungen und -konzentrationen gemäß Verfahrensschritt d) mittels mathematischer Korrelationsverfahren unter Verwendung eines oder mehrerer Kalibrierspektren einer oder mehrerer Kalibrierproben mit bekannter Elementzusammensetzung und -konzentration erfolgt.

15. Verfahren nach Anspruch 14,
**dadurch gekennzeichnet,**
daß ein Kreuzkorrelationsverfahren angewendet wird.

## Claims

1. A measuring head with a casing (32) suitable for use in a device for determining element compositions and concentrations in material samples, in particular the concentration and/or the presence of precious metals in unsorted metal scrap, by means of laser-assisted plasma emission spectroscopy employing a high-power laser (12) and a spectrometer (26), said high-power laser (12) and said spectrometer (26) being both geometrically and spatially decoupled from said measuring head (14) which has its own casing (32), said measuring head (14) including integrated beam lens system means (22) and said measuring head (14) and said integrated beam lens system means (22) being connected to said laser (12) and/or said spectrometer (26) via at least one optical beam waveguide (16, 18) included within said measuring head (14),
**characterized in**
that said casing (32) includes at least one movable spacer (34), which spacer (34), in a first position thereof, protrudes partially from the side of the casing (32) which faces the sample to be measured and, in a second position thereof, is completely within the casing (32), with the spacer (34), in its second position, releasing trigger means (38) for said laser (12).

2. The measuring head according to claim 1
**characterized in**
that said beam lens system means (22) is mounted at a defined constant distance from an exit aperture (36) of said casing (32) so that the focus of the laser beam is on the same plane as said exit aperture (36).

3. The measuring head of claim 2
**characterized in**
that said beam lens system means (22) is spaced at a distance of 25 - 40 mm from said exit aperture (36) of said casing (32).

4. The measuring head of claim 1
**characterized in**
that the high-power laser (12) comprises a frequency-multiplied Nd:YAG laser.

5. The measuring head of claims 1 or 4
**characterized in**
that a transmission switch (24) is provided between said spectrometer (26) and said beam lens system means (22).

6. The measuring head of claim 5
**characterized in**
that said transmission switch (24) consists of a Pockels cell.

7. The measuring head of one of claims 1 or 4 to 6
**characterized in**
that a detector (40) is provided within said spectrometer (26).

8. The measuring head of claim 7
**characterized in**
that said detector (40) is a multi-channel detector.

9. The measuring head of claims 7 or 8
**characterized in**
that said detector (40) comprises photodiode arrays or CCD arrays or lines containing photomultipliers.

10. A process for determining element compositions and concentrations in material samples, in particular the concentration and/or the presence of precious metals in unsorted metal scrap, by means of laser-assisted plasma emission spectroscopy, said process comprising the following steps:
a) converting part of the material sample (20) to be analysed into a plasma-like state (46) using a tunable high-power laser (12) which is focussed on said material sample (20);
b) dispersing any radiation emitted by the plasma (46) into its spectral components by means of a spectrometer (26);
(c) measuring individual spectra (42) of the plasma (46) at different laser pulses in one or several spectral ranges typical of a certain element by means of a multi-channel detector (40),
(d) calculating the element composition and concentration of the material samples on the basis of the total emission spectrum (44) measured, i.e. the sum of a plurality of measured individual spectra (42), said calculation being performed by integrating several individual measurements or spectra (42), with the pulse-to-pulse variations of the individual spectra (42) being averaged during plasma generation and the individual measurements being integrated in the multi-channel detector (40),
and the laser (12) is focussed according to process step a) by placing a measuring head (14) on the material sample (20), said measuring head (14) comprising a casing (32) in which latter beam lens system means (22) is provided at a defined constant distance from an exit aperture (38) of said casing (32) such that the focus of the laser beam is on the same plane as the exit aperture (38)
**characterized in**
that said casing (32) includes at least one movable spacer (34), said spacer (34), in a first position thereof, partially protrudes from the side of the casing (32) which faces the sample (20) to be measured and, in a second position thereof, is entirely within said casing (32), with the spacer (34), in its second position, releasing trigger means (38) for the laser source (12).

11. The process of claim 10
**characterized in**
that in process step c) the plasma emissions are filtered in time by means of an optical transmission switch (24).

12. The process of claim 11
**characterized in**
that said filtering consists in a stopping down of the wide band emission basis for a 0 - 500 nsec time period following a laser pulse and/or a stopping down of molecular and/or cluster spectra in a µsec to msec time range following a laser pulse.

13. The process of one of claims 10 to 12
**characterized in**
that at least 5, preferably 10 - 100, individual spectra (42) are evaluated in process step d).

14. The process of one of claims 10 to 13
**characterized in**
that the element compositions and concentrations are calculated according to process step d) by means of mathematical correlation processes using one or several calibration spectra of one or several calibration samples whose element composition(s) and concentration(s) are known.

15. The process of claim 14
**characterized in**
that a cross-correlation process is employed.

## Revendications

1. Tête de détection avec boîtier (32), appropriée à l'utilisation dans un dispositif pour déterminer la composition et la concentration d'éléments d'un échantillon de matériau, plus particulièrement la concentration, voire l'existence de métaux précieux dans de la ferraille désordonnée, grâce à une spectroscopie d'émission plasmatique par laser avec un laser à grande puissance (12) et un spectromètre (26), le laser à grande puissance (12) et le spectromètre (26), ce dernier possédant une tête de détection (14) à propre boîtier, étant séparés géométriquement et dans l'espace, la tête de détection (14) comportant une optique de faisceau (22) intégré et cette tête de détection (14) voire l'optique intégrée (22) étant reliées au laser (12) et/ou au spectromètre (26) par au moins un conducteur d'ondes lumineuses (16, 18) placé dans la tête de détection (14),
**caractérisée en ce que**
le boîtier (32) comporte au moins un élément d'écartement mobile (34), l'élément d'écartement (34), dans un premier état, sortant partiellement de la partie du boîtier (32) visant l'échantillon à analyser (20) et, dans un deuxième état, étant situé entièrement à l'intérieur du boîtier, ce deuxième état de l'élément d'écartement débloquant un dispositif d'activation (38) du laser (12).

2. Tête de détection suivant la revendication 1,
**caractérisée en ce que**
l'optique du faisceau (22) se situe à une distance définie et constante de l'ouverture de sortie (36) du boîtier (32), de telle sorte que le foyer du faisceau laser se trouve sur le plan de l'ouverture de sortie (36).

3. Tête de détection suivant la revendication 2,
**caractérisée en ce que**
l'optique du faisceau (22) se trouve à une distance de 25 - 40 cm de l'ouverture de sortie (36) du boîtier (32).

4. Tête de détection suivant la revendication 1,
**caractérisée en ce que** le laser à grande puissance (12) est créé à partir d'une multiplication de fréquence d'un laser Nd : YAG .

5. Tête de détection suivant la revendication 1 ou 4,
**caractérisée en ce que**
un commutateur de transmission (24) se trouve entre le spectromètre (26) et l'optique du faisceau (22).

6. Tête de détection suivant la revendication 5,
**caractérisée en ce que**
le commutateur de transmission (24) est formé d'une cellule de Pockel.

7. Tête de détection suivant l'une des revendications 1 ou 4 à 6,
**caractérisée en ce que**
le spectromètre (26) est muni d'un détecteur (40).

8. Tête de détection suivant la revendication 7,
**caractérisée en ce que**
le détecteur (40) est formé d'un détecteur multicanal.

9. Tête de détection suivant la revendication 7 ou 8,
**caractérisée en ce que**
le détecteur (40) est composé d'une barrette de photodiodes ou CCD ou de barrettes de photomultiplicateurs.

10. Procédé pour déterminer la composition et la concentration d'éléments dans des échantillons de matériaux, plus particulièrement la concentration, voire l'existence de métaux précieux dans de la ferraille désordonnée, grâce à une spectroscopie d'émission plasmatique, ce procédé englobant
a) la transposition d'une partie de l'échantillon à analyser (20) dans un état plasmatique (46) grâce à un laser de puissance (12) déclenché et focalisé sur l'échantillon de matériau (12);
b) l'analyse spectrale d'une radiation émise par le plasma (46) grâce à un spectromètre (26);
c) la mesure de chaque spectre (42) du plasma (46) par différentes impulsions laser dans une ou plusieurs régions spectrales intrinsèques aux éléments grâce à un détecteur multicanal (40);
d) le calcul de la composition et de la concentration des éléments des échantillons de matériaux à l'aide du spectre d'émission total mesuré (44), c.-à-d. de la somme d'une multitude de spectres (42), le calcul étant effectué au moyen d'une intégration de plusieurs mesures ou spectres isolés (42) et en prenant la moyenne, impulsion par impulsion, des variations de chaque spectre (42) pendant l'état plasmatique, et en effectuant l'intégration des mesures isolées dans le détecteur mutilcanal (40),
et la focalisation du laser (12) s'effectuant suivant l'opération de base a) en plaçant une tête de détection (14) sur l'échantillon du matériau (20), la tête de détection (14) étant composée d'un boîtier (32) dans lequel une optique de faisceau (22) est positionnée à une distance définie et constante de l'orifice de sortie (38) du boîtier (32), de telle sorte que le foyer du faisceau laser se trouve dans le plan de l'orifice de sortie (38),
**caractérisé en ce que**
le boîtier (32) comporte au moins un élément d'écartement mobile (34), l'élément d'écartement (34), dans un premier état, sortant partiellement de la partie du boîtier (32) visant l'échantillon à analyser (20) et, dans un deuxième état, étant situé entièrement à l'intérieur du boîtier, ce deuxième état de l'élément d'écartement débloquant un dispositif d'activation (38) de la source laser (12).

11. Procédé suivant la revendication 10,
**caractérisé en ce que**
dans l'opération de base c), une filtration temporaire des radiations plasmatiques soit effectuée au moyen d'un commutateur de transmission optique (24).

12. Procédé suivant la revendication 11,
**caractérisé en ce que**
la filtration consiste à supprimer le fond spectral à large bande des radiations pendant un intervalle de temps de 0 - 500 nsec après l'impulsion laser et/ou à supprimer les spectres moléculaires et/ou de clusters dans l'intervalle de temps des µsec. aux msec. après l'impulsion laser.

13. Procédé suivant l'une des revendications 10 à 12,
**caractérisé en ce que,**
dans l'opération de base d), au moins 5, de préférence 10 - 100 spectres isolés (42) soient évalués.

14. Procédé suivant l'une des revendications 10 à 13,
**caractérisé en ce que**
le calcul des compositions et concentrations des éléments suivant l'opération de base d) soit effectué au moyen d'analyses de corrélation mathématiques en utilisant un ou plusieurs spectres de calibration d'un ou de plusieurs échantillons de calibration à composition et concentration d'éléments connues.

15. Procédé suivant la revendication 14,
**caractérisé en ce que**
une analyse par corrélation croisée soit effectuée.
